(19) Europäisches Patentamt / European Patent Office / Office européen des brevets

(11) **EP 2 645 278 B1**

(12) **FASCICULE DE BREVET EUROPEEN**

(45) Date de publication et mention
de la délivrance du brevet:
**01.05.2019   Bulletin 2019/18**

(51) Int Cl.:
*G06F 17/30* *(2006.01)*

(21) Numéro de dépôt: **13161815.9**

(22) Date de dépôt: **28.03.2013**

(54) **Procédé d'archivage d'images et système associé**

Archivierungsverfahren von Bildern, und entsprechendes System

Image archiving method and associated system

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **30.03.2012   FR 1252891**

(43) Date de publication de la demande:
**02.10.2013   Bulletin 2013/40**

(73) Titulaire: **Orange
75015 Paris (FR)**

(72) Inventeurs:
• **Girard, Bruno
38130 ECHIROLLES (FR)**
• **Prola, Alain
38100 GRENOBLE (FR)**

(74) Mandataire: **Cabinet Plasseraud
66, rue de la Chaussée d'Antin
75440 Paris Cedex 09 (FR)**

(56) Documents cités:
**WO-A1-2008/077232**

**Description**

L'invention concerne le domaine du stockage et de l'archivage d'images à long terme, notamment pour des images médicales.

Les images médicales représentent des volumes de données de plus en plus importants à gérer. Certaines réglementations imposent un temps de stockage de ces images important pouvant aller jusqu'à 20 ans. L'accès à ces images sans perte de qualité doit être rapide et quasi immédiat les trois premières années de stockage. Le temps d'accès aux images stockées peut progressivement se ralentir au cours du temps jusqu'à in fine devenir indirect ou différé (exemple stockage sur CD ou autre support). La compression des images médicales avec perte de qualité n'est acceptée qu'après un temps bien défini par la réglementation et qui est supérieur à 3 ans. Par ailleurs, la réglementation en vigueur prévoit que les lieux de stockage des images médicales répondent à des contraintes fortes nécessitant un agrément (sécurité d'accès, un médecin doit faire partie de l'équipe de gestion, etc.....).

De nombreux problèmes se posent, notamment la problématique d'archivage à long terme des données. Les systèmes existants tels que les PACS (*Picture Archive and Communication Systems* en anglais, soit les systèmes de communication et d'archivage d'images) gèrent l'ensemble des métadonnées alphanumériques associées aux images dans des bases de données relationnelles classiques et les images médicales sous la forme de fichiers au niveau des SGF (Système de Gestion de Fichiers). Ce mécanisme entraîne rapidement des saturations d'espace disque et impose des extensions de SGF. Par ailleurs ces systèmes PACS gèrent les images médicales sans tenir compte de leur nature ou de leurs propriétés, alors que la nature de l'image telle que la modalité de capture de l'image, i.e. Scanner, IRM, Radiologie numérisée, Image ultrasonore, Médecine nucléaire, ..., la pathologie du patient ou la fréquence d'accès à l'image sont des propriétés qui impactent son processus de vieillissement.

Ainsi le temps passé par une image médicale dans la mémoire rapide doit en effet être déterminé en fonction de la modalité de capture de l'image, de la pathologie du patient à partir duquel l'image est capturée, de la fréquence d'accès de l'image ou d'autres paramètres. Par exemple, le temps passé dans la mémoire rapide par une image de fracture osseuse n'est pas le même que le temps passé par une image de tumeur cérébrale.

Il existe donc un besoin pour un mécanisme amélioré d'archivages d'images, et notamment des images médicales.

WO 2008/077232 A1 divulgue un système PACS pour élargir la fonctionnalité d'interconnexion de réseaux y comprises la migration de fichier et le changement d'état en fonction des paramètres diverses des images.

Pour remédier les inconvénients de l'art antérieur, l'invention propose d'élaborer un système de persistance des images intégrant un processus permettant d'enfouir dans des strates (des supports de mémoire physiques) plus ou moins profondes (i.e. des plus rapides vers les plus lentes) des archives d'images, notamment d'images médicales.

Plus particulièrement, l'invention concerne un procédé d'archivage d'images comprenant les étapes consistant à :

- partitionner un ensemble d'images en n classes $C_i$, $i \in [1, n]$, en fonction de règles d'ordonnancement;
- répartir les images entre une pluralité de conteneurs de stockage, chaque conteneur étant dédié au stockage d'images d'une classe donnée;
- contrôler une migration d'au moins une image d'un conteneur dédié à une première classe vers un conteneur dédié à une deuxième classe, une fonction de transformation étant appliquée à l'image lors de la migration.

L'invention concerne aussi un système d'archivage d'images comprenant :

- un ordonnanceur adapté à partitionner un ensemble d'images en n classes $C_i$, $i \in [1, n]$, en fonction de règles d'ordonnancement;
- un optimiseur d'image ;
- une pluralité de conteneurs, chaque conteneur étant dédié au stockage d'images d'une classe donnée,

dans lequel l'ordonnanceur est adapté à contrôler une migration d'au moins une image d'un conteneur dédié à une première classe vers un conteneur dédié à une deuxième classe, l'optimisateur d'image étant adapté à transformer l'image lors de sa migration.

Le procédé et le système d'archivage selon l'invention sont totalement indépendants des outils de type PACS utilisés puisqu'ils interviennent en aval de ces derniers. Le processus de migration des images d'un conteneur vers un autre conteneur au cours du cycle de stockage des images permet de réduire la place mémoire occupée par les images et de maximiser les accès aux images considérées comme les plus importantes.

Selon un mode de réalisation, le procédé comprend une étape consistant à contrôler la migration d'au moins une image d'un conteneur de la dernière classe vers une mémoire morte. Le système peut alors comprendre un robot de sauvegarde d'images sur une mémoire morte. Le procédé et le système d'archivage selon l'invention permettent de stocker sur de la mémoire morte les images au terme de leur processus de vieillissement, et d'archiver les images en fin de cycle de stockage.

Selon des modes de réalisation, le partitionnement en classes de l'ensemble des images est réalisé en fonction de l'âge des images.

L'âge d'une image peut être défini comme la durée écoulée depuis la date de génération de cette image ou depuis la date de stockage initial de cette image dans le système d'archivage.

Dans un mode de réalisation, la migration d'une image d'un premier conteneur dédié à une première classe à un deuxième conteneur dédié à une deuxième classe est effectué, après une durée de stockage dans le premier conteneur, lorsque l'âge de cette image atteint une valeur qui fait partie d'un ensemble d'au moins une valeur associé à la deuxième classe.

Ainsi, une image initialement stockée dans un premier conteneur sera migrée vers un deuxième conteneur au bout d'une certaine durée de stockage dans le premier conteneur, dès lors que l'âge de cette image aura atteint une valeur donnée qui correspond à une classe d'appartenance différente, associée au deuxième conteneur. Au cours du temps, c'est-à-dire au fur et à mesure que l'image prend de l'âge, elle est transférée de conteneur en conteneur, de manière automatisée, sur la base de l'âge courant de cette image, sans qu'une intervention humaine soit nécessaire pour réaliser cette opération de transfert.

Dans un mode de réalisation, chaque classe d'images est associée à un ensemble de valeurs possibles pour l'âge d'une image : de cette manière l'affectation d'une image à une classe est possible sur la base de son âge.

Un tel ensemble de valeurs peut être défini comme un intervalle de valeurs ou comme une valeur unique. Par exemple, un premier conteneur contient les images de moins de 5 ans, qu'un deuxième conteneur contient les images de 5 à 10 ans, un troisième contient celles de 10 à 20 ans, etc.

En particulier, les ensembles de valeurs associés aux différentes classes d'images définissent une partition de l'ensemble des valeurs d'âge possibles : ainsi, en fonction de son âge, une image sera stockée dans un conteneur déterminé sans ambiguïté et migrera automatiquement de conteneur en conteneur au fur et à mesure de son vieillissement.

Dans un mode de réalisation, l'âge de chaque image peut être pondéré par des paramètres représentatifs de la fréquence d'accès à l'image et/ou d'un paramètre représentatif du contenu de l'image. L'âge de l'image ainsi pondéré sera alors utilisé en lieu et place de l'âge effectif de l'image pour déterminer la classe d'appartenance d'une image, et donc le conteneur associé à cette classe, conteneur dans lequel l'image doit être stockée. Dans ce mode de réalisation, une image initialement stockée dans un premier conteneur sera migrée vers un deuxième conteneur au bout d'une durée déterminée de stockage dans le premier conteneur, dès lors que l'âge pondéré de cette image aura atteint une valeur donnée qui correspond à une classe d'appartenance différente, associée au deuxième conteneur.

Les règles d'ordonnancement du partitionnement définissent à un instant donné la classe d'appartenance d'une image en fonction de différents critères. Elles peuvent être définies en fonction de règles légales préétablies et/ou en fonction de paramétrages introduits par un utilisateur et/ou en fonction de propriétés liées à l'image. Le procédé et le système d'archivage selon l'invention permettent d'optimiser les performances et la fiabilité des systèmes de type PACS. En effet, le mécanisme de partitionnement de l'ensemble des images en différentes classes en fonction de certaines propriétés telles que la modalité de capture de l'image, la pathologie, la fréquence d'accès permet à l'utilisateur d'accéder plus rapidement à des images considérées comme critiques, par exemple relatives à des pathologies lourdes.

Selon un mode de réalisation, la fonction de transformation appliquée à l'image lors de la migration apporte une réduction de la taille mémoire occupée par l'image. Le système et le procédé selon l'invention permettent ainsi une décroissance rapide du volume de données à stocker une maîtrise de la taille mémoire du système de stockage. Chaque conteneur peut comprendre une mémoire de masse présentant des propriétés spécifiques adaptées à la classe correspondante pour réduire la quantité de mémoire requise pour le stockage d'une image. Par exemple, chaque conteneur peut être constitué par une partition sur une unité de stockage sécurisée de type RAID 5.

Selon une application, les images sont des images médicales. Le système et le procédé selon l'invention trouvent une application particulièrement avantageuse dans le domaine des archives médicales où le volume de données traitées devient de plus en plus important. Les règles d'ordonnancement peuvent alors déterminer la classe d'appartenance d'une image en fonction d'au moins un paramètre défini pour une pathologie associée à cette image. Un tel paramètre peut être une durée de rétention minimale requise ou un degré d'accessibilité requis pour les images associées à cette pathologie.

D'autres caractéristiques et avantages de l'invention apparaîtront à la lecture de la description détaillée ci-après, faite en référence aux figures annexées qui représentent :

- figure 1, un schéma d'un système d'archivage d'images selon l'invention ;
- figure 2, une illustration d'une interface homme-machine pour le paramétrage de la partition en classe d'âges dans un système selon l'invention ;
- figure 3, une illustration d'une interface homme-machine pour le paramétrage de critères de pondération de l'importance d'une image stockée dans un système selon l'invention ;

- figure 4, une illustration d'une interface homme-machine pour le paramétrage de l'optimisation des images stockées dans un système selon l'invention.

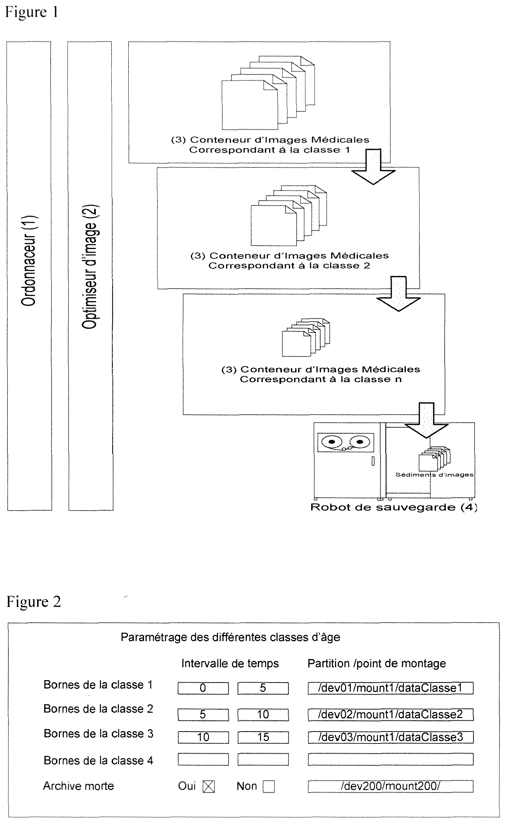

La figure 1 illustre schématiquement un système d'archivage d'images selon l'invention. Un tel système est complémentaire d'un système de type PACS qu'il vient optimiser et fiabiliser.

La figure 1 montre un ordonnanceur 1. Ce composant est responsable de la gestion du cycle de stockage des images. L'ordonnanceur 1 est adapté à accéder à un ensemble d'images E produites par des équipements de capture d'images, par exemple : scanner, IRM, radiologie numérisée, image ultrasonore, médecine nucléaire ou autre.

La figure 1 montre aussi un optimiseur d'image 2. Ce composant est adapté à réduire la place (taille mémoire) utilisée par chaque image à stocker.

La figure 1 montre encore une pluralité de conteneurs 3, chaque conteneur étant dédié au stockage d'images d'une classe donnée. Le partitionnement en différentes classes de l'ensemble des images E produites sera décrit plus en détails plus bas. Les conteneurs 3 sont dédiés à la persistance des différentes classes d'images ; ils permettent le stockage des images selon leurs classes d'appartenance sur des systèmes de mémoire de masse. Ainsi chaque conteneur 3 dédié au stockage des images d'une classe donnée pourra être représenté physiquement par une partition sur une unité de stockage sécurisée de type RAID 5 par exemple.

Chaque conteneur 3 présentera des propriétés (capacité mémoire, mémoire vive, temps d'accès...) spécifiques et donc des performances adaptées à la classe d'images correspondante, c'est-à-dire correspondant aux performances requises pour cette classe d'image. Ainsi au moins un paramètre caractérisant les performances de ce conteneur et les performances requises pour les images d'une classe d'image sera utilisé pour sélectionner le conteneur adapté à cette classe d'image. Le conteneur est par exemple sélectionné en fonction d'un temps d'accès moyen à un fichier de ce conteneur et d'un temps d'accès minimal requis pour les images de la classe d'images considérée.

La figure 1 montre en outre un robot 4 de sauvegarde d'images sur une mémoire morte. Ce robot 4 permet le stockage des images de la dernière classe d'images (images de la classe n sur la figure 1) sur de la mémoire morte telle que des bandes par exemple.

L'ordonnanceur 1 du système est adapté à contrôler le partitionnement de l'ensemble des images en différentes classes et à contrôler une migration des images d'un conteneur 3 d'une classe donnée (classe 1 par exemple) vers un conteneur 3 d'une classe de niveau sous-jacent (classe 2 par exemple) en fonction de règles d'ordonnancement. Ces règles d'ordonnancement seront décrites plus en détails plus bas ; elles sont notamment définies en fonction de règles légales préétablies et/ou en fonction de paramètres introduits par un utilisateur du système et/ou en fonction de propriétés liées à l'image.

L'optimiseur d'image 2 est adapté à transformer des fichiers associés aux images stockées lors de leur migration d'un conteneur 3 d'une classe donnée vers un conteneur 3 d'une classe de niveau sous-jacent. Cette transformation comprend une réduction de la taille de l'image. La transformation opérée par l'optimiseur d'image 2 est basée sur des règles légales préétablies et/ou sur des règles définies par l'utilisateur (le corps médical et notamment les radiologues par exemple). On utilise le terme «classe de niveau sous-jacent» car les images migrent d'une classe à l'autre en perdant progressivement de leur qualité, du fait de la transformation qui leur est appliquée à chaque changement de classe, et de leur accessibilité, du fait notamment des performances respectives des différents conteneurs associées respectivement aux différentes classes.

L'optimiseur d'image 2 peut également servir à anonymiser les images avant stockage dans un des conteneurs 3 ou leur de la migration d'un conteneur d'une classe $C_i$ vers un conteneur d'une classe de niveau sous-jacent $C_{i+1}$. Cette anonymisation consiste à supprimer dans les propriétés de l'image les références au patient pour lequel cette image a été prise.

Le mécanisme de partitionnement de l'ensemble des images par l'ordonnanceur 1 en différentes classes est maintenant décrit plus en détails.

Soit E l'ensemble des images produites par les équipements de capture d'images (scanner, IRM, ou autres) et stockées dans un système d'archivage de type PACS. Soit P(E) une partition de E en n classe d'équivalence notée $C_i$, $i \in [1, n]$. Les classes d'équivalence $C_i$, $i \in [1, n]$ représentent un partitionnement de l'ensemble des images de l'ensemble E en n classes.

On note Classe(age) la fonction qui à un âge donné d'une image retourne une classe d'équivalence $C_i$ associée, comme illustré figure 2.

Par exemple, on peut définir le partitionnement suivant :

- une première classe $C_i$ pour les images dont l'âge effectif appartient à un premier intervalle de temps $[0, T_1[$ ;
- une deuxième classe $C_2$ pour les images dont l'âge effectif appartient à un deuxième intervalle de temps $[T_1, T_2[$ ;
- une troisième classe $C_3$ pour les images dont l'âge effectif appartient à un troisième intervalle de temps $[T_2, T_3[$.

Les bornes T1, T2, T3 sont des paramètres qui peuvent être déterminés lors de la configuration du système ; ils peuvent être fixés réglementairement et/ou par l'utilisateur (un professionnel de santé par exemple) via une IHM (Interface Homme Machine) comme illustré sur la figure 2. Un support physique de persistance, i.e. un conteneur 3 ayant des caractéristiques de stockage approprié, est associé à chaque classe, par exemple via l'IHM lors de la configuration du système. Chaque conteneur 3 associé à une classe pourra présenter des propriétés spécifiques en termes de capacité de stockage et/ou de rapidité d'accès.

Par exemple, la partition P(E) de l'ensemble des images E produites peut être comme suit

- $C_1$ contenant les images dont l'âge effectif est dans l'intervalle [0, 5 ans [ ;
- $C_2$ contenant les images dont l'âge effectif est dans l'intervalle [5, 10 ans [ ;
- $C_3$ contenant les images dont l'âge effectif est dans l'intervalle [10, 15 ans [.

Il est entendu que davantage ou moins que trois classes peuvent être définies et que les bornes temporelles peuvent être différentes de l'exemple ci-dessus.

On note ainsi $f_c(O)$ la fonction caractéristique qui à chaque objet O (image) de l'ensemble E associe une classe, notamment en fonction de l'âge de l'image. Cette fonction peut, au-delà de l'âge de l'image, prendre en compte un certain nombre de paramètres qui caractérisent l'importance de l'image aux yeux de l'utilisateur du système.

Les paramètres caractérisant l'importance d'une image comprennent par exemple au moins un paramètre représentatif du contenu de l'image, c'est-à-dire de ce qui est représenté dans cette image. Par exemple, pour un système dédié aux professionnels de santé, un paramètre représentatif du contenu de l'image est la pathologie du patient sur lequel cette image a été générée : l'importance n'est en effet pas la même selon que l'image représente une tumeur cancéreuse ou bien seulement une carie dentaire.

Un autre paramètre caractérisant l'importance d'une image est la fréquence d'accès à l'image (en nombre d'accès pas an). D'autres paramètres peuvent être considérés pour caractériser l'importance de l'image stockée.

Les paramètres caractérisant l'importance d'une image peuvent être utilisés comme des critères de pondération de l'âge de l'image. Ainsi à chaque critère est associé un poids compris dans l'intervalle [0, 0.5]. Ces taux sont fixés sous la forme de paramètre de fonctionnement du système, via l'IHM par exemple.

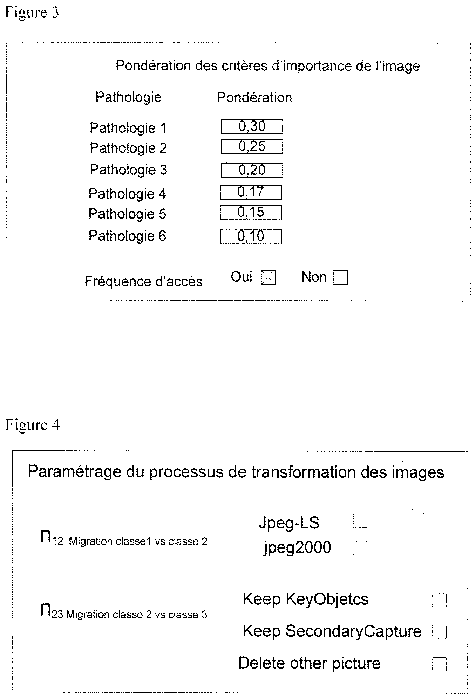

La figure 3 montre un exemple de critères de pondération de l'importance de l'image en fonction d'un paramètre représentatif du contenu de l'image qui est la pathologie. A chaque pathologie, on peut ainsi associer un poids compris entre 0% et 50%, qui contribue à déterminer le temps de rétention d'une image associée à cette pathologie dans une classe donnée. On note ainsi Patho(O) la fonction qui à un objet O de l'ensemble E associe un taux $t_x$ correspondant au taux fixé dans l'IHM comme expliqué ci-dessus en référence à la figure 3. On note Freq (O) la fonction qui à un objet O de l'ensemble E associe une fréquence d'accès déterminée dans l'IHM. La fréquence d'accès peut être définie de manière binaire (oui ou non comme illustré sur la figure 3) ou peut être un poids compris entre 0% et 100%, qui contribue également à déterminer le temps de rétention d'une image donnée dans une classe donnée.

La fonction $f_c$ peut alors être définie ainsi :

$$f_c(o) = Classe(Age(o)(1 - Patho(o) + Freq(o)))$$

La fonction $f_c$ permet donc de partitionner les images (les objets O) en classe d'équivalence $C_i$. Chaque classe $C_i$ est donc définie par les objets

$$o_j, j \in [1, Card(E)] / f_c(o_j) = C_i$$

La fonction $f_c$ utilise des règles d'ordonnancement comme définit ci-dessus ; ces règles incluent notamment des règles légales, par exemple quant à la durée de rétention minimale de certaines images dans une certaine classe, et/ou des règles définies par l'utilisateur (le corps médical dans l'application décrite), par exemple quant à l'accessibilité de certaines images du fait de leur correspondance avec une pathologie donnée, et/ou des règles définies par les propriétés de l'image, par exemple la nature de l'image (radio, scanner ou autre). Une image donnée sera placée dans une classe donnée de par son âge, mais pourra être retenue plus ou moins longtemps dans cette classe en fonction d'autres paramètres tels que la pathologie, la fréquence d'accès et la nature de l'image par exemple.

Le mécanisme d'optimisation de la taille des images par l'optimiseur d'image 2 lors de leur migration d'une classe à l'autre est maintenant décrit plus en détails.

On note $\Pi_{i,i+1}(O)$ la fonction d'optimisation/allègement mise en oeuvre lors de la migration d'un objet O (une image) d'une classe $C_i$ vers une classe $C_{i+1}$. Cette fonction est basée sur la mise en oeuvre de règles fixées par les utilisateurs (radiologues notamment) et par des règles réglementaires.

Par exemple, le radiologue pourra choisir lors de la configuration du système la/les fonction(s) de transformation des images $\Pi_{i,i+1}$ au moyen d'une IHM comme illustrée sur la figure 4. Sur la base de cette fonction de transformation $\Pi_{i,i+1}$, l'optimisateur d'image 2 assurera la conversion des images dont la migration sera ordonner par l'ordonnanceur 1 lors d'un changement de classe.

Chaque image de l'ensemble E sera stockée selon un cycle de stockage définit par le processus suivant : Une image O est produite par un équipement d'imagerie médicale approprié (radio, scanner, IRM ou autre). La date de capture de l'image est introduite dans le fichier informatique de l'image ; l'âge effectif de l'image est donc connu tout au long du cycle de stockage. L'image capturée peut en outre être paramétrée par l'utilisateur (médecin radiologue par exemple) à l'aide d'une IHM comme décrit en référence à la figure 3. L'image capturée est initialement placée dans la première classe $C_1$.

L'image O est alors stockée dans le conteneur 3 associé à la première classe $C_1$ avec une forte résolution et une possibilité d'accès rapide et fréquent.

L'image O vieillit. Au bout d'une certaine durée de stockage dans la première classe $C_1$, l'ordonnanceur 1 va déterminer que cette image O présente maintenant une fonction caractéristique $f_c(O)$ qui l'associe à la deuxième classe $C_2$. L'ordonnanceur 1 ordonne alors la migration de cette image O de la première classe $C_1$ vers la deuxième classe $C_2$. L'optimisateur d'image 2 va alors transformer l'image O, en modifiant son fichier informatique de stockage conformément à la fonction d'optimisation $\Pi_{i,i+1}(O)$ définie lors de la configuration du système, pour obtenir un fichier requérant moins de place mémoire. L'image O est alors placée dans le conteneur 3 associé à la deuxième classe $C_2$. Cette opération est répétée plusieurs fois lors du vieillissement de l'image O qui migre ainsi d'une classe $C_i$ vers une classe sous jacente $C_{i+1}$ au cours du cycle de stockage. Il est entendu que le système peut comprendre plus de trois classes et que les figures ne limitent pas l'invention à l'usage stricte de trois classes.

L'image O continue de vieillir. Après avoir migrée d'une classe à l'autre comme décrit ci-dessus, l'image O peut finalement être stockée par un robot 4 dans une mémoire morte. L'ordonnanceur 1 ordonne alors la migration de cette image O de la dernière classe $C_n$ vers la mémoire morte. Une trace de l'image O demeure, même après un stockage de très longue durée, et au besoin, cette image O est récupérable avec ses éléments essentiels depuis la mémoire morte.

Le système et le procédé selon l'invention permettent ainsi une sédimentation des images O produites, notamment pour des images médicales. Au cours du cycle de stockage des images médicales, ces dernières vont passer d'une strate à une autre jusqu'à ne devenir que des « sédiments » en étant transformées lors du processus. Lors de l'enfouissement des images d'une strate à l'autre - d'un conteneur à un autre, ces dernières seront plus ou moins transformées afin d'être allégées par un processus spécifique inhérent à leurs propriétés intrinsèques telles que la modalité de capture d'image, la pathologie du patient pour lequel l'image a été capturée, ou d'autres propriétés. A terme, seules les parties dites essentielles des images archivées demeurent, i.e. pour les images médicales les « keys objects » (objets clés) s'ils existent, les « secondary captures » (prises secondaires) si elles existent, ou sinon les images compressées avec pertes. Dans ce but, l'optimiseur d'image permet d'effectuer une réduction de taille d'image en compressant moins les parties essentielles, et en appliquant au contraire une compression plus importante sur les parties non essentielles. A l'ultime stade du processus de stockage, ces images, comparables à des sédiments, seront archivées sur de la mémoire morte, i.e. bandes ou autres supports. Tout comme le processus de sédimentation laisse la mémoire fossile des animaux ou végétaux, ce mécanisme d'archivage permettra de conserver une ultime emprunte de l'image médicale archivée sur de la mémoire morte.

Le système et le procédé selon l'invention peuvent être mis en oeuvre dans des centres de données (data center) dédiés à l'hébergement d'images médicales ou d'autre type de données. Le système et procédé selon l'invention permettent à un opérateur offrant un service de stockage de réduire l'espace mémoire consommé en mémoire de masse et, à la fin du cycle de stockage, de ne conserver que les données considérées comme essentielles des objets archivés sur de la mémoire morte.

L'invention a été décrite en référence à des modes de réalisations particuliers qui ne sont pas limitatifs. Notamment, tout autre partitionnement que celui décrit en trois classes avec des intervalles de temps de 5 ans peut être envisagé. En outre, tout autre paramétrage que ceux décrit en référence aux figures 3 et 4 peut être envisagé pour utiliser d'autres paramètres de pondération lors du partitionnement des objets archivés. La description a été faite en relation avec l'archivage d'images médicales, mais d'autres applications peuvent être envisagées, comme par exemple le stockage de données d'autre nature que les images médicales, comme par exemple des documents administratifs scannés, les très vieux ouvrages scannés du fond documentaire national, les archives de films de l'INA etc.

**Revendications**

1. Procédé d'archivage d'images comprenant les étapes consistant à :

- partitionner un ensemble d'images P(E) en n classes $C_i$, $i \in [1, n]$, en fonction de règles d'ordonnancement;
- répartir les images entre une pluralité de conteneurs (3) de stockage, chaque conteneur étant dédié au stockage d'images d'une classe donnée ($C_i$) ;
- contrôler une migration d'au moins une image d'un conteneur dédié à une première classe ($C_i$) vers un conteneur dédié à une deuxième classe ($C_{i+1}$), une fonction de transformation ($\Pi_{i,i+1}$) étant appliquée à l'image lors de la migration.

2. Procédé d'archivage selon la revendication 1, comprenant ou outre une étape consistant à contrôler une migration d'au moins une image d'un conteneur de la dernière classe ($C_n$) vers une mémoire morte.

3. Procédé d'archivage selon l'une quelconque des revendications précédentes, dans lequel le partitionnement P(E) de l'ensemble des images de l'ensemble (E) en n classes $C_i$ comprend un partitionnement en fonction de l'âge des images.

4. Procédé d'archivage selon la revendication 1 ou 3, dans lequel la migration d'une image d'un premier conteneur dédié à une première classe ($C_i$) à un deuxième conteneur dédié à une deuxième classe ($C_{i+1}$) est effectué, après une durée de stockage dans le premier conteneur, lorsque l'âge de ladite image atteint une valeur qui fait partie d'un ensemble d'au moins une valeur associé à la deuxième classe.

5. Procédé d'archivage selon l'une quelconque des revendications précédentes, dans lequel les règles d'ordonnancement définissent la classe d'appartenance d'une image en fonction d'une fréquence d'accès à l'image et/ou de d'au moins un paramètre représentatif du contenu de l'image.

6. Procédé d'archivage selon l'une quelconque des revendications précédentes, dans lequel les règles d'ordonnancement définissent la classe d'appartenance d'une image en fonction de règles légales préétablies et/ou en fonction de paramétrages introduits par un utilisateur et/ou en fonction de propriétés liées à l'image.

7. Procédé d'archivage selon l'une quelconque des revendications précédentes, dans lequel la fonction de transformation ($\Pi_{i,i+1}$) apporte une réduction de la taille mémoire occupée par l'image.

8. Procédé d'archivage selon l'une des revendications précédentes, dans lequel les images (E) sont des images médicales, les règles d'ordonnancement définissant la classe d'appartenance d'une image en fonction d'au moins un paramètre défini pour une pathologie associée à cette image.

9. Procédé d'archivage selon la revendication 8, dans lequel le paramètre est une durée de rétention minimale requise ou un degré d'accessibilité requis pour les images associées à cette pathologie.

10. Système d'archivage d'images comprenant :

- un ordonnanceur (1) adapté à partitionner un ensemble d'images P(E) en n classes $C_i$, $i \in [1, n]$, en fonction de règles d'ordonnancement ;
- un optimiseur d'image (2) ;
- une pluralité de conteneurs (3), chaque conteneur étant dédié au stockage d'images d'une classe donnée ($C_i$), dans lequel l'ordonnanceur (1) est adapté à contrôler une migration d'au moins une image d'un conteneur dédié à une première classe ($C_i$) vers un conteneur dédié à une deuxième classe ($C_{i+1}$), l'optimisateur d'image (2) étant adapté à transformer l'image lors de sa migration.

11. Système d'archivage d'images selon la revendication 10, comprenant en outre un robot (4) de sauvegarde d'images sur une mémoire morte et des moyens pour contrôler une migration d'au moins une image d'un conteneur de la dernière classe ($C_n$) vers la mémoire morte.

12. Système d'archivage d'images selon l'une quelconque des revendications 10 à 11, dans lequel chaque conteneur (3) présente des performances adaptées à la classe (Ci) d'images à laquelle il est dédié.

**Patentansprüche**

1. Bildarchivierungsverfahren, umfassend die folgenden Schritte:

- Partitionieren einer Gesamtheit von Bildern P(E) in n Klassen $C_i$, $i \in [1,n]$ in Abhängigkeit von Scheduling-Regeln;
- Verteilen der Bilder auf eine Vielzahl von Speichercontainern (3), wobei jeder Container zur Speicherung von Bildern einer gegebenen Klasse ($C_i$) bestimmt ist;
- Kontrollieren einer Migration mindestens eines Bildes aus einem Container, der für eine erste Klasse ($C_i$) bestimmt ist, zu einem Container, der für eine zweite Klasse ($C_{i+1}$) bestimmt ist, in Abhängigkeit von einer Transformation ($\Pi_{i,i+1}$), die bei der Migration auf das Bild angewandt wird.

2. Archivierungsverfahren nach Anspruch 1, umfassend ferner einen Schritt, der darin besteht, eine Migration mindestens eines Bildes aus einem Container der letzten Klasse ($C_n$) zu einem Nur-Lese-Speicher zu kontrollieren.

3. Archivierungsverfahren nach einem der vorhergehenden Ansprüche, bei dem die Partitionierung P(E) der Gesamtheit der Bilder der Gesamtheit (E) in n Klassen $C_i$ eine Partitionierung in Abhängigkeit vom Alter der Bilder umfasst.

4. Archivierungsverfahren nach Anspruch 1 oder 3, bei dem die Migration eines Bildes aus einem ersten Container, der für eine erste Klasse ($C_i$) bestimmt ist, in einen zweiten Container, der für eine zweite Klasse ($C_{i+1}$) bestimmt ist, nach einer Speicherdauer in dem ersten Container erfolgt, wenn das Alter des Bildes einen Wert erreicht, der zu einer Gesamtheit mindestens eines der zweiten Klasse zugeordneten Wertes gehört.

5. Archivierungsverfahren nach einem der vorhergehenden Ansprüche, bei dem die Scheduling-Regeln die Zugehörigkeitsklasse eines Bildes in Abhängigkeit von einer Zugriffshäufigkeit auf das Bild und/oder von mindestens einem für den Inhalt des Bildes repräsentativen Parameter definieren.

6. Archivierungsverfahren nach einem der vorhergehenden Ansprüche, bei dem die Scheduling-Regeln die Zugehörigkeitsklasse eines Bildes in Abhängigkeit von vorgegebenen gesetzlichen Regeln und/oder in Abhängigkeit von Parametrierungen, die von einem Benutzer eingegeben werden, und/oder in Abhängigkeit von mit dem Bild zusammenhängenden Eigenschaften definieren.

7. Archivierungsverfahren nach einem der vorhergehenden Ansprüche, bei dem die Transformationsfunktion ($\Pi_{i,i+1}$) zu einer Verringerung des von dem Bild belegten Speicherplatzes führt.

8. Archivierungsverfahren nach einem der vorhergehenden Ansprüche, bei dem die Bilder (E) medizinische Bilder sind, wobei die Scheduling-Regeln die Zugehörigkeitsklasse eines Bildes in Abhängigkeit von mindestens einem Parameter definieren, der für eine diesem Bild zugeordnete Erkrankung definiert ist.

9. Archivierungsverfahren nach Anspruch 8, bei dem der Parameter eine geforderte Mindestvorhaltezeit oder ein geforderter Zugänglichkeitsgrad für die dieser Erkrankung zugeordneten Bilder ist.

10. Bildarchivierungssystem, umfassend:

    - einen Scheduler (1), der geeignet ist, eine Gesamtheit von Bildern P(E) in Abhängigkeit von Scheduling-Regeln in n Klassen $C_i$, $i \in [1, n]$ zu partitionieren;
    - einen Bildoptimierer (2);
    - eine Vielzahl von Containern (3), wobei jeder Container zur Speicherung von Bildern einer gegebenen Klasse ($C_i$) bestimmt ist,
    wobei der Scheduler (1) geeignet ist, eine Migration mindestens eines Bildes aus einem Container, der für eine erste Klasse ($C_i$) bestimmt ist, zu einem Container, der für eine zweite Klasse ($C_{i+1}$) bestimmt ist, zu kontrollieren, wobei der Bildoptimierer (2) geeignet ist, das Bild bei seiner Migration zu transformieren.

11. Bildarchivierungssystem nach Anspruch 10, umfassend ferner einen Roboter (4) zum Sichern von Bildern in einem Nur-Lese-Speicher und Mittel, um eine Migration mindestens eines Bildes aus einem Container der letzten Klasse ($C_n$) in den Nur-Lese-Speicher zu kontrollieren.

12. Bildarchivierungssystem nach einem der Ansprüche 10 bis 11, bei dem jeder Container (3) geeignete Leistungen für die Klasse (Ci) von Bildern, für die er bestimmt ist, aufweist.

**Claims**

1. Image archiving method comprising the steps consisting in:

   - partitioning a set of images P(E) into n classes $C_i$, $i \in :[l, n]$, according to sequencing rules;
   - distributing the images between a plurality of storage containers (3), each container being dedicated to the storage of images of a given class ($C_i$) ;
   - controlling a migration of at least one image from a container dedicated to a first class ($C_i$) to a container dedicated to a second class ($C_{i+l}$), a transformation function ($\Pi_{i,i+l}$) being applied to the image during the migration.

2. Archiving method according to Claim 1, furthermore comprising a step consisting in controlling a migration of at least one image from a container of the last class ($C_n$) to a non-volatile memory.

3. Archiving method according to any one of the preceding claims, in which the partitioning P(E) of the set of the images of the set (E) into n classes $C_i$ comprises a partitioning according to the age of the images.

4. Archiving method according to either of Claims 1 and 3, in which the migration of an image from a first container dedicated to a first class ($C_i$) to a second container dedicated to a second class ($C_{i+l}$) is carried out, after a period of storage in the first container, when the age of the said image reaches a value which belongs to a set of at least one value associated with the second class.

5. Archiving method according to any one of the preceding claims, in which the sequencing rules define the class to which an image belongs as a function of a frequency of access to the image and/or of at least one parameter representative of the content of the image.

6. Archiving method according to any one of the preceding claims, in which the sequencing rules define the class to which an image belongs according to pre-established legal rules and/or according to settings introduced by a user and/or according to properties associated with the image.

7. Archiving method according to any one of the preceding claims, in which the transformation function ($\Pi_{i,i+l}$) provides a reduction in the memory size occupied by the image.

8. Archiving method according to one of the preceding claims, in which the images (E) are medical images, the sequencing rules defining the class to which an image belongs as a function of at least one parameter defined for a pathology associated with this image.

9. Archiving method according to Claim 8, in which the parameter is a required minimum retention time or a degree of accessibility required for the images associated with this pathology.

10. Image archiving system comprising:

    - a sequencer (1) designed to partition a set of images P(E) into n classes $C_i$, $i \in : [l, n]$, according to sequencing rules;
    - an image optimizer (2);
    - a plurality of containers (3), each container being dedicated to the storage of images of a given class ($C_i$), in which the sequencer (1) is designed to control a migration of at least one image from a container dedicated to a first class ($C_i$) to a container dedicated to a second class ($C_{i+l}$), the image optimizer (2) being designed to transform the image during its migration.

11. Image archiving system according to Claim 10, furthermore comprising a robot (4) for saving images in a non-volatile memory and means for controlling a migration of at least one image from a container of the last class ($C_n$) to the non-volatile memory.

12. Image archiving system according to any one of Claims 10 to 11, in which each container (3) exhibits performance characteristics adapted to the class ($C_i$) of images to which it is dedicated.

Figure 1

(3) Conteneur d'Images Médicales
Correspondant à la classe 1

(3) Conteneur d'Images Médicales
Correspondant à la classe 2

(3) Conteneur d'Images Médicales
Correspondant à la classe n

Sédiments d'Images

Robot de sauvegarde (4)

Ordonnaceur (1)

Optimiseur d'image (2)

Figure 2

| Paramétrage des différentes classes d'âge | | | |
|---|---|---|---|
| | Intervalle de temps | | Partition /point de montage |
| Bornes de la classe 1 | 0 | 5 | /dev01/mount1/dataClasse1 |
| Bornes de la classe 2 | 5 | 10 | /dev02/mount1/dataClasse2 |
| Bornes de la classe 3 | 10 | 15 | /dev03/mount1/dataClasse3 |
| Bornes de la classe 4 | | | |
| Archive morte | Oui ⊠ Non ☐ | | /dev200/mount200/ |

Figure 3

| Pondération des critères d'importance de l'image | |
| --- | --- |
| Pathologie | Pondération |
| Pathologie 1 | 0,30 |
| Pathologie 2 | 0,25 |
| Pathologie 3 | 0,20 |
| Pathologie 4 | 0,17 |
| Pathologie 5 | 0,15 |
| Pathologie 6 | 0,10 |
| Fréquence d'accès | Oui ☒   Non ☐ |

Figure 4

Paramétrage du processus de transformation des images

$\Pi_{12}$ Migration classe1 vs classe 2

Jpeg-LS ☐

jpeg2000 ☐

$\Pi_{23}$ Migration classe 2 vs classe 3

Keep KeyObjetcs ☐

Keep SecondaryCapture ☐

Delete other picture ☐

**EP 2 645 278 B1**

## RÉFÉRENCES CITÉES DANS LA DESCRIPTION

**Documents brevets cités dans la description**

- WO 2008077232 A1